# EUROPÄISCHE PATENTSCHRIFT

(11) **EP 0 680 314 B1**
(45) Veröffentlichungstag und Bekanntmachung des Hinweises auf die Patenterteilung: **07.05.1997**
(21) Anmeldenummer: 94905062.9
(22) Anmeldetag: 14.01.1994
(51) Int. Cl.: A61K 7/50, C11D 1/835

(54) **DETERGENSGEMISCHE MIT VERBESSERTEN AVIVAGEEIGENSCHAFTEN**
DETERGENT MIXTURES WITH IMPROVED BRIGHTENING PROPERTIES
MELANGES DETERGENTS POSSEDANT DE MEILLEURES PROPRIETES D'AVIVAGE

(30) Priorität: 23.01.1993 DE 4301820; 25.02.1993 DE 4305726
(43) Veröffentlichungstag der Anmeldung: 08.11.1995
(73) Patentinhaber: Henkel Kommanditgesellschaft auf Aktien, 40191 Düsseldorf (DE)
(72) Erfinder: HENSEN, Hermann, D-42781 Haan (DE); TESMANN, Holger, D-41363 Jüchen (DE); KAHRE, Jörg, D-40789 Monheim (DE); GOEBELS, Dagmar, D-40591 Düsseldorf (DE)
(86) Internationale Anmeldenummer: EP9400097
(87) Internationale Veröffentlichungsnummer: WO9416677

(56) Entgegenhaltungen:
- EP-A- 0 094 118
- EP-A- 0 538 762
- WO-A-92/07543

## Beschreibung

### Gebiet der Erfindung

Die Erfindung betrifft Detergensgemische enthalten Alkyl- und/oder Alkenyloligoglykoside, Esterquats und Ölkörper, Haarbehandlungsmittel, die diese Gemische enthalten sowie die Verwendung der Gemische zur Herstellung von Haarbehandlungsmitteln.

### Stand der Technik

Alkyloligoglykoside, insbesondere Alkyloligoglucoside stellen nichtionische Tenside dar, die infolge ihrer ausgezeichneten Detergenseigenschaften und hohen ökotoxikologischen Verträglichkeit zunehmend an Bedeutung gewinnen. Herstellung und Verwendung dieser Stoffe sind gerade in letzter Zeit in einer Reihe von Übersichtsartikeln dargestellt worden, von denen stellvertretend die Veröffentlichungen von H.Hensen in **Skin Care Forum, 1, (Okt.1992),** D.Balzer und N.Ripke in **Seifen-Öle-Fette-Wachse 118, 894 (1992)** und B.Brancq in **Seifen-Öle-Fette-Wachse 118, 905 (1992)** genannt werden sollen.

Auch die gemeinsame Verwendung von Alkyloligoglucosiden und kationischen Polymeren in Haarbehandlungsmitteln ist aus einer Reihe von Druckschriften bekannt.

So wird in der **US 4 668 422** (Henkel) eine Rezeptur für ein Schaumbad offenbart, die C_{9/11}- und C_{12/13}-Alkyloligoglucoside, Betaine, Aminoxide, Perlglanzmittel und kationische Copolymere von Acrylamid und Dimethyldiallylammoniumchlorid enthalten. Aus der **JP-A 01/144 497** (Shiseido) sind Haarshampoos bekannt, die neben C_{8/20}-Alkyloligoglykosiden, kationische Polymere, quartäre Ammoniumverbindungen und anionische Tenside enthalten. In der **DE-A 30 18 600** (L'Oreal) wird in Beispiel 16 ein Haarshampoo vorgeschlagen, das neben C_{8/20}-Alkyloligoglucosiden und Saponinen kationische Stärke enthält. Aus der **EP-A 0 094 118** sind Zusammensetzungen bekannt, die Alkyloligoglucoside, quartäre Ammoniumverbindungen und Polysiloxanöle enthalten. Gegenstand der **EP-A1 0 337 354** sind schließlich milde Tensidmischungen enthaltend Alkyloligoglucoside und kationische Polymere.

Gemische von Alkyloligoglykosiden und quartären Ammoniumverbindungen bzw. kationischen Polymeren sind somit hinlänglich bekannt und als vorteilhafte Bestandteile von besonders milden Haarshampoos und Schaumbädern ausführlich beschrieben. Demgegenüber weisen weder Alkyloligoglucoside, noch bekannte monomere kationische Tenside oder deren Mischungen überraschenden Vorteile, insbesondere im Hinblick auf die Haaravivage auf. Alkyloligoglykoside alleine tragen beispielsweise kaum zu einer Verbesserung der Naß- und Trockenkämmbarkeit von Haaren bei, das Leistungsvermögen quartärer Ammoniumverbindungen reicht an das kationischer Polymere ebenfalls nicht heran. Der Zusatz von Alkyloligoglykosiden zu bekannten monomeren kationischen Tensiden führt eher zu einer Verschlechterung der Haaravivage. Für den Einsatz in Haarbehandlungsmitteln sind Mischungen von Alkyloligoglykosiden und quartären Ammoniumverbindungen somit eher ungeeignet.

Trotz dieser ungünstigen Ausgangslage besteht bei den Herstellern kosmetischer Produkte infolge der hohen ökotoxikologischen Verträglichkeit der Produkte ein dringendes Bedürfnis nach Haarbehandlungsmitteln auf Basis von Alkyloligoglykosiden und kationischen monomeren Tensiden, wobei die Avivageleistung dieser Gemische jedoch zumindest den Produkten des Stands der Technik entsprechen sollte. Die Aufgabe der Erfindung hat somit darin bestanden, derartige Tensidmischungen unter Umgehung der aufgezeigten Nachteile zu entwickeln.

### Beschreibung der Erfindung

Gegenstand der Erfindung sind Detergensgemische mit verbesserten Avivageeigenschaften, enthaltend
a) Alkyl- und/oder Alkenyloligoglykoside der Formel **(I)**, 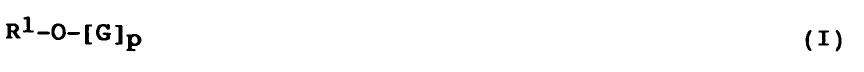 in der R¹ für Alkyl- und/oder Alkenylreste mit 6 bis 22 Kohlenstoffatomen, G für einen Zuckerrest mit 5 oder 6 Kohlenstoffatomen und p für Zahlen von 1 bis 10 steht,
b) Esterquats der Formel **(II)**, 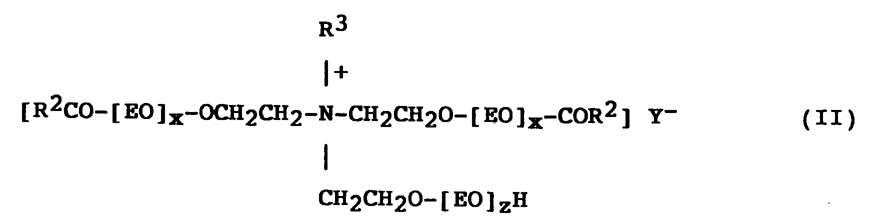 in der R²CO für einen aliphatischen, gegebenenfalls hydroxysubstituierten Acylrest mit 12 bis 22 Kohlenstoffatomen und 0 oder 1 Doppelbindung, R⁷ für eine Methylgruppe oder eine Polyethylenglycoletherkette mit 1 bis 5 Ethylenoxideinheiten, x, y und z für 0 oder in Summe für Zahlen von 1 bis 5 und Y für Halogen, Alkylsulfat oder Alkylphosphat steht, und
c) Ölkörper.

Überraschenderweise wurde gefunden, daß der Zusatz von Ölkörpern die Haaravivage von Mischungen aus Alkyl- und/oder Alkenyloligoglykosiden und Esterquats signifikant verbessert.

### Alkyl- und/oder Alkenyloligoglykoside

Alkyl- und Alkenyloligoglykoside stellen bekannte Stoffe dar, die nach den einschlägigen Verfahren der präparativen organischen Chemie erhalten werden können. Stellvertretend für das umfangreiche Schrifttum sei hier auf die Schriften **EP-A1-0 301 298** und **WO 90/3977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**.

Die Indexzahl p in der allgemeinen Formel **(I)** gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 6 bis 11, vorzugsweise 8 bis 10 Kohlenstoffatomen ableiten. Typische Beispiele sind Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3).

Der Alkyl- bzw. Alkenylrest R¹ kann sich ferner auch von primären Alkoholen mit 12 bis 22, vorzugsweise 12 bis 16 Kohlenstoffatomen ableiten. Typische Beispiele sind Laurylalkohol, Myristylalkohol, Cetylalkohol, Palmoleylalkohol, Stearylalko- hol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Esterquats

Esterquats der Formel **(II)** stellen technische Gemische von gegebenenfalls ethoxylierten Mono- und Difettsäuretriethanolaminestern dar, die mit Ethylenoxid, Alkylhalogeniden, Dialkylsulfaten oder Dialkylphosphaten quaterniert sind. Auch bei ihnen handelt es sich um bekannte Stoffe. Vorzugsweise werden Esterquats eingesetzt, die sich von Palm-, Kokos- oder Talgfettsäuren einer Iodzahl im Bereich 0 bis 40 ableiten und einen Veresterungsgrad von 1, 5 bis 1,9 aufweisen. Der Ethoxylierungsgrad kann 0 oder 1 bis 5, vorzugsweise 1 bis 3 betragen. Aus anwendungstechnischen Gründen besonders geeignet sind Esterquats der Formel **(II)**, in der R³ für eine Methylgruppe oder eine Polyethylenglycolkette mit 1 bis 3 Ethylenoxideinheiten und Y für Chlorid oder Methylsulfat steht. Besonders bevorzugt ist ein methylquaternierter Dipalm-, Dikokos- oder Ditalgfettsäuretriethanolaminester (Dehyquart^{(R)} AU36, Pulcra/Barcelona).

### Ölkörper

Als Ölkörper kommen beispielsweise fette Öle, Fettsäureester, Guerbetalkohole, Dialkylcyclohexane, Dialkylether, Paraffinkohlenwasserstoffe und Siliconöle in Betracht.

Unter **fetten Ölen** sind synthetische, vorzugsweise jedoch natürliche Triglyceride zu verstehen, die einen hohen Anteil ungesättigter Fettsäuren enthalten und eine Iodzahl im Bereich von 100 bis 170 aufweisen. Typische Beispiele sind Raps- und Sonnenblumenöl neuer Züchtung, Erdnußöl, Baumwollsaatöl, Korianderöl, Olivenöl, Meadowfoamöl, Avocadoöl, Mandelöl und Nussöl.

Als **Fettsäureester** kommen Verbindungen der Formel **(III)** in Betracht, 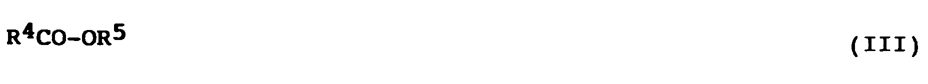 in der R⁴CO für einen aliphatischen, linearen oder verzweigten, gegebenenfalls hydroxysubstituierten Acylrest mit 6 bis 22 Kohlenstoffatomen und R⁵ für einen Alkyl- und/oder Alkenylrest mit 1 bis 22 Kohlenstoffatomen steht.

Typische Beispiel sind Ester von Fettsäuren, ausgewählt aus der Gruppe die von Capronsäure, Caprylsäure, Isononansäure, Caprylsäure, Undecansäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Ricinolsäure, 12-Hydroxystearinsäure, Arachinsäure, Gadoleinsäure, Behensäure und Erucasäure sowie deren technischen Gemischen gebildet wird, mit Alkoholen, ausgewählt aus der Gruppe, die von Methanol, Ethanol, n-Propanol, i-Proponal, Butanol, Pentanol, Hexanol, Heptanol, Octanol, 2-Ethylhexanol, Caprinalkohol, Laurylalkohol, Myristylalkohol, Cetylalkohol, Stearylalkohol, Isostearylalkohol, Oleylalkohol, Elaidylalkohol, Ricinolylalkohol, Behenylalkohol und Erucylalkohol sowie deren technischen Gemischen gebildet wird. Besonders bevorzugt ist der Einsatz von Estern der Isononansäure und der C_{16/18}-Fettsäuren mit i-Propylalkohol oder Cetylstearylalkohol.

Unter **Guerbetalkoholen** sind Verbindungen der Formel **(IV)** zu verstehen, 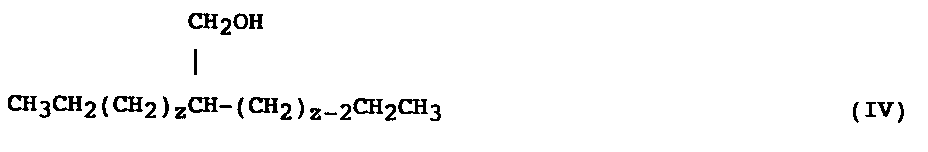 in der z für Zahlen von 6 bis 12 steht.

Hierbei handelt es sich um primäre, verzweigte Alkohole, die auf bekannte Weise durch basenkatalysierte Dimerisierung von Fettalkoholen hergestellt werden. Typische Beispiele stellen 2-Hexyldecanol, 2-Octyldodecanol und 2-Decyltetradecanol dar.

Bei den **Dialkylcyclohexanen** handelt es sich um bekannte Stoffe, die durch einschlägige Verfahren der präparativen organischen Chemie erhalten werden können. Ein Beispiel zu ihrer Herstellung besteht beispielsweise darin, Dialkylaromaten (ortho-/meta-/para-Xylol) aus der BTX-Fraktion des Erdöls einer katalytischen Hydrierung zu unterwerfen.

Die in Betracht kommenden **Dialkylcyclohexane** folgen der Formel **(V)**, 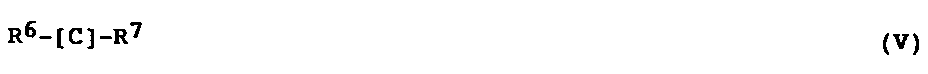 in der R⁶ und R⁷ unabhängig voneinander für Alkylreste mit 1 bis 12 Kohlenstoffatomen und C für einen Cyclohexylrest steht. Typische Beispiele sind Dimethylcyclohexan, Diethylcyclohexan, Methylethylcyclohexan, Dipropylcyclohexan, Di-n-butylcyclohexan, Di-tert.butylcyclohexan, Di-2-ethylhexylcyclohexan und insbesondere Di-n-octylcylohexan.

Unter **Dialkylethern** sind Verbindungen der Formel **(VI)** zu verstehen, 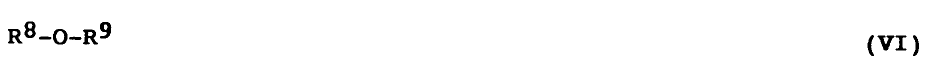 in der R⁸ und R⁹ unabhängig voneinander für Alkylreste mit 6 bis 22 Kohlenstoffatomen stehen.

Auch hierbei handelt es sich um bekannte Stoffe, die nach den einschlägigen Verfahren der präparativen organischen Chemie erhalten werden können. Verfahren zu ihrer Herstellung, beispielsweise durch Kondensation von Fettalkoholen in Gegenwart von p-Toluolsulfonsäure, sind beispielsweise aus **Bull.Soc. Chim.France, 333 (1949), DE-A1 40 39 950** (Hoechst) sowie **DE-A1 41 03 489** (Henkel) bekannt. Aus anwendungstechnischer Sicht sind symmetrische Dialkylether bevorzugt, die 6 bis 12 Kohlenstoffatomen in den Alkylresten aufweisen. Ein besonders rasches Spreitvermögen weisen Dialkylether der Formel **(VI)** auf, in der R¹² und R¹³ für Octyl- und/oder 2-Ethylhexylreste stehen. Die im Sinne der Erfindung besonders bevorzugten Dialkylether sind somit Di-n-octylether und Di-2-ethylhexylether.

Unter **Paraffinkohlenwasserstoffen** sind bekannte technische Alkangemische zu verstehen, wie sie üblicherweise als Ölkörper in kosmetischen Produkten eingesetzt werden. Vorzugsweise handelt es sich hierbei um dünnflüssige Paraffine, die eine Dichte von 0,81 bis 0,875 aufweisen.

Der Begriff **Siliconöl** umfaßt schließlich die Gruppe der Polyorganosiloxane, bei denen Siliciumatome über Sauerstoffatome ketten- und/oder netzartig verbunden und die restlichen Valenzen durch Alkylgruppen abgesättigt sind. Typische Beispiele für Siliconöle, die als geeignete Ölkörper Verwendung finden können, sind in **Parfüm Kosmet. 67, 232 (1986)** aufgelistet.

Üblicherweise können die Alkyl- und/oder Alkenyloligoglykoside und die Esterquats im Gewichtsverhältnis 1 : 3 bis 3 : 1, vorzugsweise 1 : 2 bis 2 : 1 eingesetzt werden. Die gleichen Verhältnisse empfehlen sich auch für den Einsatz von Alkyl- und/oder Alkenylglykosiden und Ölkörpern.

### Wäßrige Haarbehandlungsmittel

Ein weiterer Gegenstand der Erfindung betrifft wäßrige Haarbehandlungsmittel, enthaltend
15 bis 30 Gew.-% Alkyl- und/oder Alkenyloligoglykoside der Formel **(I)**,
40 bis 70 Gew.-% Esterquats der Formel **(II)** und
15 bis 30 Gew.-% Ölkörper
- jeweils bezogen auf den Feststoffanteil - sowie gegebenenenfalls weitere übliche Hilfs- und Zusatzstoffe.

Unter Haarbehandlungsmittel sind in diesem Zusammenhang insbesondere Haarspülungen und Haarshampoos sowie "two-in-one" -Kombinationen beider Produkte zu verstehen, die den Haaren Kämmbarkeit und Glanz verleihen sowie die antistatische Aufladung herabsetzen sollen.

### Hilfs- und Zusatzstoffe

Neben den Alkyl- und/oder Polyglykosiden und den Esterquats können die erfindungsgemäßen Detergensgemische sowie die Harbehandlungsmittel in untergeordneten Mengen weitere **Tenside** enthalten. Typische Beispiele sind Fettalkoholpolycolethersulfate, Monoglyceridsulfate, Ethercarbonsäuren, Alkylamidobetaine oder Einweißfettsäurekondensate.

Als Hilfs- und Zusatzstoffe kommen fermer auch **Emulgatoren** wie etwa alkoxylierte Fettalkohole oder Sorbitanester in Betracht. Als **Überfettungsmittel** können Substanzen wie beispielsweise polyethoxylierte Lanolinderivate, Lecithinderivate und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon sowie Elektrolyte wie Kochsalz und Ammoniumchlorid. Unter **biogenen Wirkstoffen** sind beispielsweise Pflanzenextrakte, Eiweißhydrolysate und Vitaminkomplexe zu verstehen. Gebräuchliche Filmbildner sind beispielsweise Polyvinylpyrrolidon, Vinylpyrrolidon-Vinylacetat-Copolymerisate, Polymere der Acrylsäurereihe, quaternäre Cellulose-Derivate und ähnliche Verbindungen. Als **Konservierungsmittel** eignen sich beispielsweise Phenoxyethanol, Formaldehydlösung, Parabene, Pentadiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 0 bis 50, vorzugsweise 5 bis 40 Gew.-% bezogen auf den Feststoffgehalt der Gesamtmischung ausmachen.

### Gewerbliche Anwendbarkeit

Die erfindungsgemäßen Detergensgemische verbessern die Naß- und Trockenkämmbarkeit von Haaren, verringern die elektrostatische Aufladung, verleihen Haaren einen angenehmen Weichgriff und einen seidigen Glanz; desweiteren wird ein vorteilhaftes Verdickungsverhalten beobachtet. Die Erfindung bietet somit erstmals eine Lehre an, nach der man Haarbehandlungsmittel mit zufriedenstellenden Eigenschaften auf Basis von Alkyloligoglucosiden und Esterquats herstellen kann.

Ein weiterer Gegenstand der Erfindung betrifft daher die Verwendung der erfindungsgemäßen Detergensgemische zur Herstellung von wäßrigen Haarbehandlungsmitteln, in denen sie in Mengen von 1 bis 100, vorzugsweise 5 bis 70 Gew.-% - bezogen auf den Feststoffgehalt der Mittel - enthalten sein können.

Die folgenden Beispiele sollen den Gegenstand der Erfindung näher erläutern, ohne ihn darauf einzuschränken.

### Beispiele

### I. Rezepturen

Rezeptur A ist erfinndungsgemäß, die Rezepturen X, Y und Z dienen dem Vergleich.

**Tab.1**

| Zusammensetzung der untersuchten Rezepturen Alle Zahlenangaben als Gew.-% | | | | |
|---|---|---|---|---|
| **Komponenten** | **Rezepturen** | | | |
| | X | Y | Z | A |
| C_{12/16}-Kokosalkyloligoglyucosid Plantaren^{R} 1200, Fa. Henkel KGaA, Düsseldorf/FRG | 2 | 0 | 2 | 2 |
| Dipalmfettsäuretriethanolaminester, methylquaterniert, Methylsulfatsalz | 0 | 0 | 0 | 4 |
| Dioctylcyclohexan | 0 | 0 | 0 | 2 |
| Cetyl-trimethylammoniumchlorid Dehyquart^{R} E, Fa. Henkel KGaA, Düsseldorf/FRG | 0 | 4 | 4 | 0 |

Alle Rezepturen enthalten ferner 3 Gew.-% Emulgator (Cetylstearylalkohol), 0,3 Gew.-% Konservierungsmittel und ad 100 Gew.-% Wasser.

### II. Trocken- und Naßkämmbarkeitsuntersuchungen

a) Trockenkämmbarkeit
   Die Trockenkämmbarkeit wurde unter Zulassung der elektrostatischen Aufladung untersucht. Es wurde eine relative Luftfeuchtigkeit von 20 % eingestellt. Die Konditionierungszeit betrug 12 h bei 30°C. Die Messung erfolgte über den Ladungsabgriff an einem doppelten Faraday-Käfig nach Ausführung von 10 Kämmungen. Der Fehler bei den Messungen betrug im Mittel 2,5 %, die statistische Sicherheit lag bei mindestens 99,9 %. Die Ergebnisse der Kämmarbeiten sind in Tab.2 dargestellt.
b) Naßkämmbarkeit
   Die Naßkämmbarkeit wurde an braunem Haar (Alkinco #6634, Strähnenlänge 12 cm, Strähnenmasse 1 g) untersucht. Nach der Nullmessung wurden die Strähnen mit 100 ml der Formulierungen A bis J bzw. U, V und W getränkt. Nach einer Einwirkzeit von 5 min wurden die Strähnen 1 min unter fließendem Wasser (1 l/min, 38°C) ausgespült. Die Strähnen wurden erneut vermessen und mit der Nullmessung verglichen. Der Fehler bei den Messungen betrug im Mittel 2 %, die statistische Sicherheit lag bei mindestens 99 %. Die Ergebnisse sind ebenfalls in Tab.2 dargestellt.

Eine ausführliche Beschreibung der Meßmethoden befindet sich in **J.Soc.Cosm.Chem.,** **24****, 782 (1973**.

**Tab.2**

| Trocken- und Naßkämmbarkeit | | | | | | | |
|---|---|---|---|---|---|---|---|
| Bsp. | R | Trockenkämmbarkeit [mJ] | | | Naßkämmbarkeit [mJ] | | |
| | | vor. | nach. | Diff. | vor. | nach. | Diff. |
| 1 | A | 5,3 | 4,6 | 0,7 | 70,1 | 12,0 | 58,1 |
| V1 | X | 6,0 | 5,9 | 0,1 | 63,4 | 31,5 | 31,9 |
| V2 | Y | 5,8 | 5,3 | 0,5 | 59,2 | 19,2 | 40,0 |
| V3 | Z | 4,8 | 4,5 | 0,3 | 57,9 | 18,1 | 39,8 |
| Legende: R = Rezeptur vor. = vorher nach. = nachher Diff. = Differenz | | | | | | | |

## Patentansprüche

1. Detergensgemische mit verbesserten Avivageeigenschaften, enthaltend
a) Alkyl- und/oder Alkenyloligoglykoside der Formel **(I)**, 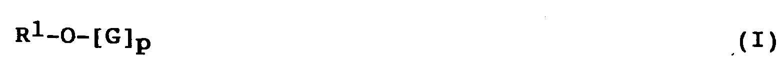 in der R¹ für Alkyl- und/oder Alkenylreste mit 6 bis 22 Kohlenstoffatomen, G für einen Zuckerrest mit 5 oder 6 Kohlenstoffatomen und p für Zahlen von 1 bis 10 steht,
b) Esterquats der Formel **(II)**, 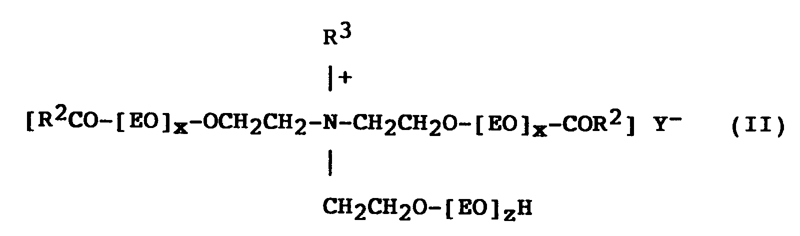 in der R²CO für einen aliphatischen, gegebenenfalls hydroxysubstituierten Acylrest mit 12 bis 22 Kohlenstoffatomen mit 0 oder 1 Doppelbindung, R³ für eine Methylgruppe oder eine Polyethylenglycoletherkette mit 1 bis 5 Ethylenoxideinheiten, x, y und z für 0 oder in Summe für Zahlen von 1 bis 5 und Y für Halogen, Alkylsulfat oder Alkylphosphat steht, und
c) Ölkörper.

2. Detergensgemische nach Anspruch 1, **dadurch gekennzeichnet**, daß sie Alkylglucoside der Formel **(I)** enthalten, in der R¹ für einen Alkylrest mit 12 bis 16 Kohlenstoffatomen, G für einen Glucoserest und p für Zahlen von 1 bis 3 steht.

3. Detergensgemische nach den Ansprüchen 1 und 2, **dadurch gekennzeichnet**, daß sie Ölkörper enthalten, ausgewählt aus der Gruppe, die von fetten Ölen, Fettsäureestern, Guerbetalkoholen, Dialkylethern, Dialkylcyclohexanen, Paraffinkohlenwasserstoffen und Siliconölen gebildet wird.

4. Detergensgemische nach den Ansprüchen 1 bis 3, **dadurch gekennzeichnet**, daß sie die Alkyl- und/oder Alkenyloligoglykoside und die Esterquats im Gewichtsverhältnis 1 : 3 bis 3 : 1 enthalten.

5. Detergensgemische nach den Ansprüchen 1 bis 4, **dadurch gekennzeichnet**, daß sie die Alkyl- und/oder Alkenyloligoglykoside und die Ölkörper im Gewichtsverhältnis 1 : 3 bis 3 : 1 enthalten.

6. Wäßrige Haarbehandlungsmittel, enthaltend
15 bis 30 Gew.-% Alkyl- und/oder Alkenyloligoglykoside der Formel **(I)**,
40 bis 70 Gew.-% Esterquats der Formel **(II)**, und
15 bis 30 Gew.-% Ölkörper
- jeweils bezogen auf den Feststoffanteil - sowie gegebenenenfalls Emulgatoren, Konservierungsmittel und weitere übliche Hilfsstoffe.

7. Verwendung von Detergensgemischen nach den Ansprüchen 1 bis 6 zur Herstellung von wäßrigen Haarbehandlungsmitteln.

## Claims

1. Detergent mixtures having improved conditioning properties containing
a) alkyl and/or alkenyl oligoglycosides corresponding to formula **(I)**: 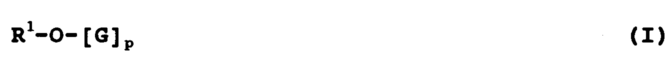 in which R¹ represents alkyl and/or alkenyl radicals containing 6 to 22 carbon atoms, G is a sugar unit containing 5 or 6 carbon atoms and p is a number of 1 to 10,
b) esterquats corresponding to formula **(II)**: 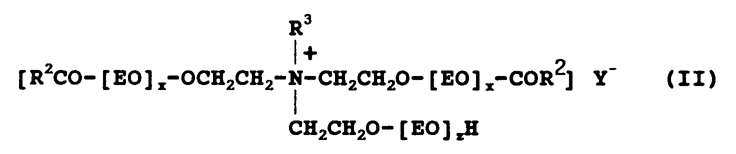 in which R²CO is an aliphatic, optionally hydroxy-substituted acyl radical containing 12 to 22 carbon atoms and 0 or 1 double bond, R³ is a methyl group or a polyethylene glycol ether chain containing 1 to 5 ethylene oxide units, x, y and z = 0 or, together, stand for numbers of 1 to 5 and Y is halogen, alkyl sulfate or alkyl phosphate, and
c) oils.

2. Detergent mixtures as claimed in claim 1, **characterized in that** they contain alkyl glucosides corresponding to formula **(I)** in which R¹ is an alkyl radical containing 12 to 16 carbon atoms, G is a glucose unit and p is a number of 1 to 3.

3. Detergent mixtures as claimed in claims 1 and 2, **characterized in that** they contain oils selected from the group consisting of fatty oils, fatty acid esters, Guerbet alcohols, dialkyl ethers, dialkyl cyclohexanes, paraffin hydrocarbons and silicone oils.

4. Detergent mixtures as claimed in claims 1 to 3, **characterized in that** they contain the alkyl and/or alkenyl oligoglycosides and the esterquats in a ratio by weight of 1:3 to 3:1.

5. Detergent mixtures as claimed in claims 1 to 4, **characterized in that** they contain the alkyl and/or alkenyl oligoglycosides and the oils in a ratio by weight of 1:3 to 3:1.

6. Aqueous hair treatment preparations containing
15 to 30% by weight of alkyl and/or alkenyl oligoglycosides corresponding to formula **(I)**,
40 to 70% by weight of esterquats corresponding to formula **(II)** and
15 to 30% by weight of oils
- based in each case on the solids content - and optionally emulsifiers, preservatives and other typical auxiliaries.

7. The use of the detergent mixtures claimed in claims 1 to 6 for the production of aqueous hair treatment preparations.

## Revendications

1. Mélanges de détergents à propriétés d'avivage améliorées contenant :
a) des alkyl et/ou alcényloligoglycosides de formule (I),
R¹-O-[G]ₚ (I)
où R¹ représente un radical alkyle et/ou alcényle de 6 à 22 atomes de carbone, G est un radical sucre avec 5 ou 6 atomes de carbone et p représente des nombres de 1 à 10,
b) des esterquats de formule (II) 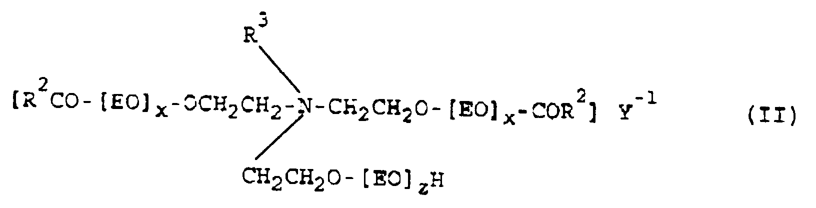 où R²CO représente un radical acyle aliphatique, le cas échéant substitué par un hydroxy de 12 à 22 atomes de carbone avec 0 ou 1 double liaison, R³ est un groupe méthyle ou une chaîne poly(éther d'éthylèneglycol) avec 1 à 5 unités oxyde d'éthylène, x, y et z représentent 0, ou leur somme représente les nombres de 1 à 5 et Y est un halogène, sulfate d'alkyle ou phosphate d'alkyle et,
c) des huiles.

2. Mélanges de détergents selon la revendication 1,
caractérisés en ce qu'
ils contiennent des alkylglucosides de formule (I) où R¹ est un radical alkyle de 12 à 16 atomes de carbone, G est un radical glucose et p est un nombre de 1 à 3.

3. Mélanges de détergents selon les revendications 1 et 2,
caractérisés en ce qu'
ils contiennent des huiles choisies dans le groupe des huiles grasses, des esters d'acides gras, des alcools de Guerbet, des éthers dialkyliques, des dialkylcyclohexanes, des hydrocarbures paraffiniques et des huiles de silicone.

4. Mélanges de détergents selon les revendications 1 à 3,
caractérisés en ce qu'
ils contiennent les alkyl- et/ou alcényloligoglycosides et les esterquats en rapport pondéral 1:3 à 3:1.

5. Mélanges de détergents selon les revendications 1 à 4,
caractérisés en ce qu'
ils contiennent les alkyl- et/ou alcényloligoglycosides et les huiles en des rapports pondéraux de 1:3 à 3:1.

6. Produits aqueux de soins capillaires contenant,
15 à 30 % en poids d'alkyl- et/ou alcényloligoglycosides de formule (I),
40 à 70 % en poids d'esterquats de formule (II), et
15 à 30 % d'huiles,
par rapport respectivement à la teneur en solides ainsi que le cas échéant par rapport à des émulsionnants, des conservateurs et d'autres additifs usuels.

7. Utilisation de mélanges de détergents selon les revendications 1 à 6, pour produire des produits aqueux de soins capillaires.
